# FASCICULE DE BREVET EUROPEEN

(11) **EP 2 559 342 B1**
(45) Date de publication et mention de la délivrance du brevet: **13.11.2019**
(21) Numéro de dépôt: 12180725.9
(22) Date de dépôt: 16.08.2012
(51) Int. Cl.: A23L 17/50, A23L 33/12, A23K 50/80, A01K 61/54, A01K 67/033

(54) **Procédé d'amélioration de la valeur nutritive d'huîtres, par stabulation en présence de microalgues**
Verfahren zur Verbesserung des Nährwerts von Austern, durch Zucht in Zuchtanlagen in Gegenwart von Mikroalgen
Method for improving the nutritional value of oysters, by rearing in the presence of microalgae

(30) Priorité: 16.08.2011 FR 1157359
(43) Date de publication de la demande: 20.02.2013
(73) Titulaire: Fermentalg, 33500 Libourne (FR)
(72) Inventeur: Calleja, M. Pierre, 33000 Bordeaux (FR)
(74) Mandataire: Be IP

(56) Documents cités:
- US-A- 4 080 930
- DELAPORTE M ET AL: "Incorporation and modification of dietary fatty acids in gill polar lipids by two bivalve species Crassostrea gigas and Ruditapes philippinarum", COMPARATIVE BIOCHEMISTRY AND PHYSIOLOGY. PART A, MOLECULAR AND INTEGRATIVE PHYSIOLOGY, ELSEVIER SCIENCE, NEW YORK, NY, US, vol. 140, no. 4, 1 avril 2005 (2005-04-01) , pages 460-470, XP004926202, ISSN: 1095-6433, DOI: 10.1016/J.CBPB.2005.02.009
- RIVERO-RODRIGUEZ ET AL: "The effect of microalgal diets on growth, biochemical composition, and fatty acid profile of Crassostrea corteziensis (Hertlein) juveniles", AQUACULTURE, ELSEVIER, AMSTERDAM, NL, vol. 263, no. 1-4, 31 janvier 2007 (2007-01-31), pages 199-210, XP005867072, ISSN: 0044-8486, DOI: 10.1016/J.AQUACULTURE.2006.09.038
- MILKE L M ET AL: "Comparison of early life history stages of the bay scallop, Argopecten irradians: Effects of microalgal diets on growth and biochemical composition", AQUACULTURE, ELSEVIER, AMSTERDAM, NL, vol. 260, no. 1-4, 29 septembre 2006 (2006-09-29), pages 272-289, XP027903486, ISSN: 0044-8486 [extrait le 2006-09-29]
- KING WAI FAN, FENG CHEN: "Production of High-Value Products by Marine Microalgae Thraustochytrids", REFEREX, 1 septembre 2006 (2006-09-01), pages 293-323, XP040425876,

## Description

L'invention concerne les domaines de l'ostréiculture et de l'alimentation.

Un mollusque filtreur est un mollusque à coquille tel qu'une moule, un pétoncle, une coquille Saint-Jacques, une huître, une coque ou un ormeau, qui filtre l'eau de mer et fixe les nutriments et autres principes actifs présents dans les microalgues, c'est-à-dire qu'ils retiennent dans leurs tissus une partie desdits nutriments ou principes actifs contenus dans les microalgues. Dans certains cas, les nutriments s'accumulent dans les tissus et s'y concentrent.

L'invention se rapporte donc à un procédé d'amélioration de la chair des huîtres, et plus particulièrement de leur valeur nutritive, consistant à les enrichir en certains nutriments, notamment en acides gras polyinsaturés et/ou en caroténoïde(s), à l'aide de microalgues, suivant la revendication 1.

Selon ce procédé, les huîtres sont mises en stabulation dans de l'eau de mer dans laquelle des microalgues d'intérêt sont ajoutées, riches en acides gras polyinsaturés et/ou en caroténoïde(s), à une concentration qui permet aux mollusques de fixer dans leur tissus certains desdits acides gras polyinsaturés et/ou caroténoïde(s).

Le ou les acide(s) gras polyinsaturé(s) visé(s) par l'invention est/sont choisi(s) parmi l'acide éicosapentaénoïque (EPA), l'acide docosahexaénoïque (DHA) et/ou l'acide arachidonique (ARA).

Les caroténoïdes visés par l'invention sont l'astaxanthine, la lutéine, la fucoxanthine, la zéaxanthine et/ou la violaxanthine.

Les huîtres sont des bivalves marins reconnus depuis l'Antiquité pour leurs qualités nutritionnelles et diététiques.

A ce titre, elles sont riches en protéines et pauvres en sucres, graisses et cholestérol, ce qui en fait un aliment de choix pour de nombreux régimes alimentaires.

L'huître comprend notamment de nombreux oligoéléments et minéraux essentiels tels que du zinc (6,5 mg/100 g), de l'iode (0,06 mg/100g), du sélénium (0,06 mg/100g), du manganèse (1 mg/100 g), du fer (5,8 mg/100 g), ainsi que du calcium, du magnésium, du potassium, du fluor et du cuivre. Elle est également riche en vitamines B et D, ainsi que, dans une moindre mesure, en vitamines C et E, lesquelles vitamines sont préservées par le fait que l'huître se consomme habituellement crue.

Elle comprend également des acides gras polyinsaturés à hauteur de 336 mg/100g de chair crue d'huître en moyenne, en particulier environ 146 mg d'EPA/100g de chair crue d'huître et 71 mg de DHA/100g de chair crue d'huître [*Composition nutritionnelle des produits aquatiques -* http://www.nutraqua.com].

Cependant, cette teneur en acides gras polyinsaturés reste relativement modeste au regard de certains poissons ou crustacés.

La production française d'huîtres s'élève actuellement à environ 130 000 tonnes par année, ce qui représente l'essentiel de la production européenne (environ 90%). La majeure partie de cette production concerne l'huître creuse, appelée parfois huître japonaise, dont le nom latin est *Crassostrea gigas.* L'huître plate, dont le nom latin est *Ostrea edulis,* est également produite ponctuellement, par exemple en Bretagne ou sur le bassin d'Arcachon.

La culture de l'huître comprend généralement trois grandes étapes :
- Le captage: les larves (le naissain) qui sont planctoniques, se fixent sur des collecteurs en tuiles, bois, ardoise, fer ou plastique, dans les zones maritimes où les courants littoraux les concentrent. Lorsque le naissain a grossi sur le collecteur, il faut le détroquer : il s'agit de décoller les petites huîtres ainsi formées.
- L'élevage (ou la croissance) des huîtres : la plupart du temps, cet élevage est effectué par une culture en poche. On place les jeunes huîtres dans des poches en plastique installées sur des tables en fer immergées près des côtes, lesquelles poches sont régulièrement retournées pour éviter une croissance en longueur, et éventuellement vidées afin de calibrer les huîtres et les remettre dans des poches nettoyées. Toutefois, il s'avère qu'une huître peut grandir jusqu'à présenter une taille importante sans pour autant être un produit de qualité, du fait de la maigreur de sa chair. Il semble en effet que la croissance de l'huître et l'engraissement de sa chair ne soient pas forcément concomitants et une étape supplémentaire est souvent nécessaire afin de faire accumuler à l'animal les réserves alimentaires nécessaires à son engraissement. C'est l'affinage.
- L'affinage : il peut être pratiqué en eau libre ou dans des bassins d'affinage, généralement appelés "claires", sur des huîtres dont la croissance est ou non achevée. Par affinage en eau libre, on entend un affinage effectué dans un biotope soumis sans contrainte à l'action de la mer. Il s'agit de l'ensemble des parcs établis dans les estuaires, les baies, les rades ou les lagunes en communication permanente avec la mer (par opposition aux sites où les mouvements de l'eau peuvent être au moins partiellement contrôlés). En eau libre, l'affinage peut se produire en accompagnant la croissance. Dans ce cas, l'engraissement dépend largement de la densité de population d'huîtres. Alternativement, les huîtres adultes peuvent être affinées dans des claires dans les derniers mois avant leur commercialisation afin d'atteindre les qualités gustatives et un volume de chair appropriés. En France, lors de l'affinage, un autre critère de qualité commerciale des huîtres peut s'ajouter à l'engraissement : c'est le verdissement, phénomène produit par une algue phytoplanctonique, la *Navicule bleue.*

C'est au cours de cet affinage que l'huître acquiert une typicité propre à sa région de production.

La région de Marennes-Oléron, qui détient une grande surface de claires en milieu naturel, peu profondes, creusées dans un sol argileux et alimentées naturellement en eau de mer, est devenue la principale région d'affinage des huîtres en France.

Ainsi, une huître née et élevée en Bretagne, en Normandie ou en Méditerranée peut, dans les deux derniers mois précédant sa consommation, être affinée à Marennes-Oléron.

Cet affinage permet d'obtenir un produit de forme homogène, dont la chair est présente en quantité suffisante sous une forme translucide à blanc, parfois teintée de vert, ayant une odeur marine agréable, un goût affiné, une saveur équilibrée (salée, puis sucrée), une consistance molle à un peu ferme, et une longueur en bouche moyenne.

Souvent consommées crues, les huîtres font l'objet d'un suivi sanitaire très strict qui implique la surveillance de la qualité des eaux dans lesquelles elles sont affinées.

En effet, les huîtres peuvent donner lieu à des troubles digestifs, notamment lorsqu'elles contiennent trop de bactéries et plus particulièrement lorsqu'elles ont filtré de l'eau contenant une trop grande concentration de microalgues dont certaines sont toxiques. Les contaminations par les microalgues sont souvent liées à la pollution de l'eau de mer, en particulier celle des bassins d'affinage, par des nitrates d'origine continentale ou bien à des phénomènes de marée de faible coefficient ne permettant pas un renouvellement suffisant en eau de mer des parcs à huîtres. Les huîtres concentrent alors des toxines appelées phycotoxines qui peuvent être toxiques pour le consommateur.

Cette pollution "naturelle" des zones d'élevage est toutefois réversible si la qualité des eaux s'améliore, car l'huître filtre en permanence l'eau de mer et rejette progressivement les toxines produites par ces algues (principalement des dinoflagellées et diatomées toxiques) lorsque celles-ci disparaissent.

La fermeture préventive des zones de production est parfois la seule possibilité d'éviter les intoxications alimentaires liées aux microalgues.

Jusqu'à présent, ce sont essentiellement les propriétés gastronomiques des huîtres qui ont guidé les ostréiculteurs dans leurs techniques d'élevage, et non l'obtention d'une valeur nutritionnelle particulière des huîtres.

Dans ce contexte, l'affinage a essentiellement pour but d'améliorer les propriétés organoleptiques des huîtres et d'accentuer leur typicité, afin de proposer au consommateur un produit de qualité homogène d'une année sur l'autre. En particulier, l'affinage n'a pas pour but de garantir la composition nutritionnelle de l'huître.

A l'opposé de cette démarche, l'inventeur a mis au point un procédé d'affinage ayant pour but d'améliorer la valeur nutritionnelle des huîtres, notamment en acides gras polyinsaturés et/ou en caroténoïde(s).

Allant à l'encontre des précautions habituellement prises dans la pratique traditionnelle des ostréiculteurs, l'inventeur a élevé des huîtres en présence de concentrations importantes de différentes espèces de microalgues d'intérêt.

De manière surprenante, l'inventeur a pu constater qu'une stabulation des huîtres en présence de microalgues d'intérêt, loin de rendre les huîtres toxiques, pouvait conduire à une amélioration de la valeur nutritionnelle de celles-ci. En particulier, il a pu observer que la stabulation des huîtres en présence de microalgues riches en acides gras polyinsaturés et/ou en caroténoïde(s), permettait la fixation par les huîtres d'une teneur importante de ces acides gras et/ou caroténoïde(s).

L'inventeur a donc pu établir que, dans la mesure où les microalgues d'intérêt n'étaient pas productrices de toxines, il était possible d'ajouter ces microalgues dans les bassins de stabulation et d'affiner les huîtres dans de bonnes conditions, de sorte à obtenir une augmentation significative de leur teneur en différents nutriments, sans modifier nécessairement leurs propriétés organoleptiques.

L'inventeur a pu constater, en outre, que les huîtres pouvaient également fixer d'autres nutriments, tels que des oligoéléments, des vitamines, des agents antioxydants ou des acides aminés essentiels, ainsi que des polysaccharides, des polyosides ou des glycosides, ces derniers pouvant avoir un effet sur le goût ou la texture des huîtres.

L'invention a donc pour objet un procédé d'enrichissement d'huîtres en acides gras polyinsaturés et/ou en caroténoïde(s).

Par acides gras polyinsaturés, on vise le DHA (acide docosahexaénoïque, C22:6 ω3), l'EPA (acide éicosapentaénoïque, C20:5 ω3) et/ou l'ARA (acide arachidonique, C20 :4 ω6). Ces acides gras essentiels sont bien connus pour leurs effets bénéfiques sur le système nerveux et en matière de prévention des maladies cardiovasculaires [Von Schacky, S. (2006) A Review of Omega-3 Ethyl Esters for Cardiovascular Prevention and Treatment of Increased Blood Triglyceride Levels. Vasc Health Risk Manag. 2(3): 251-262]. Par caroténoïdes, l'invention vise l'astaxanthine, la lutéine, la fucoxanthine, la zéaxanthine et/ou la violaxanthine. Outre leurs propriétés de pigmentation (du rouge au vert foncé, en passant par l'orange et le jaune), les caroténoïdes sont également connus pour leurs propriétés antioxydantes.

L'inventeur a découvert, de façon surprenante, qu'il était possible d'augmenter la teneur des huîtres en acides gras polyinsaturés et/ou en caroténoïde(s) de façon significative, en mettant les mollusques filtreurs en stabulation dans une eau de mer comprenant une concentration appropriée de microalgues d'intérêt contenant de tels acides gras et/ou caroténoïde(s). Il est apparu en effet que les huîtres, avaient la capacité de fixer ces acides gras et/ou caroténoïde(s) et de les accumuler dans leurs propres tissus. Le document XP004926202 (Delaporte Maryse et al. : "Incorporation and modification of dietary fatty acids in gill polar lipids by two bivalve species Crassostrea gigas and Ruditapes philippinarum", COMPARATIVE BIOCHEMISTRY AND PHYSIOLOGY. PART A, MOLECULAR AND INTEGRATIVE PHYSIOLOGY, ELSEVIER SCIENCE, NEW YORK, NY, US, vol. 140, no. 4, 1 avril 2005 (2005-04-01), pages 460-470) décrit une mise sous diète contrôlée en type de microalgues de mollusques filtreurs juvéniles afin d'évaluer l'impact de ces diètes sur la composition en lipides des branchies de ces mollusques.

Ainsi, selon un des principaux aspects de l'invention, le procédé d'enrichissement d'huîtres comprend les étapes de la revendication indépendante 1.

La stabulation correspond à un séjour des huîtres dans une eau de mer dont la qualité est contrôlée.

Un bassin d'eau de mer est de préférence une construction permettant de maintenir un volume d'eau de mer relativement constant au cours du temps, même en présence de la marée. Le cas échéant, la marée peut permettre le renouvellement d'une partie du volume d'eau de mer présent dans le bassin, ce qui peut nécessiter, selon les circonstances, de devoir rajouter régulièrement les microalgues d'intérêt dans le bassin, si la concentration de celles-ci diminue. Avantageusement, le volume d'eau de mer contenu dans le bassin d'eau de mer est de 1m³ à 100m³. Le bassin d'eau de mer peut également présenter la forme d'un plan d'eau naturel, présentant par exemple une superficie allant de 100 m² à 1 ou 2 ha.

Par microalgues d'intérêt, on entend des microalgues qui sont sélectionnées et cultivées au préalable dans des appareils de culture permettant d'obtenir des cultures concentrées. Les techniques de culture sont connues de l'homme du métier. Les microalgues peuvent être produites en mode autotrophe, hétérotrophe ou mixotrophe selon l'espèce. Le plus souvent, le mode de culture est choisi de sorte à pouvoir obtenir des microalgues ayant une teneur suffisante en acides gras polyinsaturés [Bahnweg, G. (1979) Studies on the physiology of Thraustochytriales I. Growth requirements and nitrogen nutrition of Thraustochytrium spp.,Schizochytrium sp., Japonochytrium sp., Ulkenia spp. and Labyrinthuloides spp. Veroff. Inst. Meeresforsch. Bremerh. 17: 245-268] [Ceron Garcia, M.C. et al. (2005) Mixotrophic growth of the microalga Phaeodactylum tricornutum: Influence of different nitrogen and organic carbon sources on productivity and biomass composition. Process Biochemistry 40(1):297-305].

Il a été déterminé qu'une teneur d'au moins 2 %, préférentiellement d'au moins 5 %, et plus préférentiellement d'au moins 10 % en acides gras polyinsaturés en poids sec sur la matière sèche des microalgues, était souhaitable pour que le processus de transfert des acides gras des microalgues au mollusque filtreur soit suffisant pour obtenir un enrichissement du mollusque filtreur sur une période donnée.

De même, une teneur d'au moins 1%, de préférence au moins 2%, plus préférentiellement au moins 3%, encore plus préférentiellement au moins 3,5% en poids sec sur la matière sèche de microalgues d'au moins un caroténoïde, était souhaitable pour obtenir un enrichissement du mollusque filtreur sur une période donnée.

Outre ses qualités nutritionnelles en tant qu'antioxydant, la fixation du caroténoïde dans la chair d'une huître va permettre de colorer cette chair. Cela peut être particulièrement avantageux dans le cas de la fucoxanthine, qui va contribuer au verdissement de l'huître. Par ailleurs, il est possible, via certains caroténoïdes de couleur orangée ou rosée, d'apporter une teinte saumonée ou rosée à l'huître. Enfin, concernant les moules, les caroténoïdes orangés peuvent permettre de renforcer la couleur orangée de leur chair.

De plus, il est également possible de modifier le goût des huîtres en apportant une note fruitée (telle que d'abricot) et/ou fleurie grâce à l'apport d'au moins un caroténoïde. Enfin, la texture de la chair des huîtres est également rendue plus moelleuse grâce à cet apport.

Selon un mode de réalisation préféré, les microalgues d'intérêt sont sélectionnées à l'aide d'un procédé de culture en mode mixotrophe dans l'obscurité mais avec un apport de lumière discontinu ou variable au cours du temps et dont l'intensité en micromoles de photons varie d'une amplitude égale ou supérieure à 10 µmol. m⁻².s⁻¹ à raison de plusieurs fois par heure. Un tel procédé de culture permet d'obtenir des microalgues enrichies en acides gras polyinsaturés et/ou en caroténoïdes. Un tel procédé de culture est décrit, par exemple, dans la demande WO 2012/035262.

Selon l'invention, l'huître est enrichie en acides gras polyinsaturés et en caroténoïdes. En effet, de manière avantageuse, la présence du caroténoïde dans la chair du mollusque filtreur (de l'huître) permet de stabiliser les acides gras polyinsaturés.

Avantageusement, les microalgues d'intérêt peuvent consister en un mélange de différentes espèces ayant les propriétés définies ci-dessus.

De préférence, les microalgues sont apportées sous la forme de cultures concentrées, préalablement réalisées dans les règles de l'art, afin notamment de s'assurer que celles-ci ne sont pas contaminées par des microalgues toxiques. A cet égard, un contrôle préalable de la qualité de l'eau de mer est recommandé pour s'assurer que des microalgues toxiques indésirables ne sont pas présentes avant d'ajouter les microalgues d'intérêt.

Il a également été déterminé que les microalgues devaient de préférence se trouver dans ledit bassin d'eau de mer à une concentration optimale d'au moins 10⁴ cellules/mL pour obtenir un enrichissement rapide des huîtres en acide(s) gras polyinsaturé(s) et/ou en caroténoïde(s). Cette concentration peut cependant varier selon les espèces de microalgues introduites dans le bassin d'eau de mer.

Le procédé d'enrichissement selon l'invention sera réalisé juste avant la commercialisation des huîtres, plus particulièrement dans les 1 à 10 semaines précédant la consommation desdites huîtres. La durée du procédé d'enrichissement selon l'invention sera avantageusement comprise entre 2 jours et 10 semaines, de préférence 2 à 60 jours, plus préférentiellement 1 à 5 semaines.

Le procédé d'enrichissement selon l'invention est appliqué à la culture des huîtres, soit durant l'élevage (la croissance) des huîtres, soit de manière préférée, durant l'étape d'affinage.

Dans ce cas, le procédé d'enrichissement est un procédé d'affinage d'une huître de consommation comportant un enrichissement en acides gras polyinsaturés et/ou en caroténoïdes comportant les étapes de la revendication indépendante 1.

Le procédé d'affinage d'une huître peut être réalisé sur une période allant de 2 jours à 10 semaines, de préférence de 2 à 60 jours, et plus préférentiellement encore de 1 à 5 semaines. Cette durée est généralement conforme à celle d'un affinage traditionnel, sans ajout de microalgues d'intérêt.

De préférence, les microalgues d'intérêt mises en œuvre dans les procédés selon l'invention sont sélectionnées parmi les espèces du genre suivant : *Schizochytrium, Thraustochytrium, Odontella, Phaeodactylum, Nanochloris, Crypthecodinium, Monodus, Nannochloropsis, Isochrysis, Euglena, Cyclotella, Nitzschia, Aurantiochytrium, Scenedesmus* et/ou *Tetraselmis.* Plus préférentiellement, les microalgues d'intérêt sont choisies parmi les espèces des genres *Schizochytrium, Thraustochytrium, Odontella, Phaeodactylum, Nanochloris, Crypthecodinium, Monodus, Nannochloropsis, Isochrysis, Euglena, Cyclotella, Nitzschia, Aurantiochytrium, Scenedesmus,* et encore plus préférentiellement parmi les espèces des genres *Schizochytrium, Thraustochytrium, Odontella, Phaeodactylum, Nanochloris, Crypthecodinium, Monodus, Nannochloropsis.*

Selon le type d'enrichissement que l'on désire, il est possible en effet de choisir une ou plusieurs microalgues parmi celles indiquées ci-avant. En particulier, afin d'obtenir un enrichissement en DHA, il peut être avantageux d'introduire des microalgues d'intérêt des genres *Schizochytrium, Odontella, Isochrysis, Cyclotella* ou *Nitzschia.* De même, afin d'obtenir un enrichissement particulièrement avantageux en EPA, les microalgues suivantes pourront être introduites : *Odontella, Monodus, Isochrysis, Euglena* ou *Cyclotella.* Enfin, la microalgue *Euglena* permet d'obtenir un enrichissement en ARA.

Concernant les caroténoïdes, les microalgues suivantes sont particulièrement avantageuses : *Schizochytrium* (astaxanthine), *Nitzschia* (fucoxanthine), *Aurantiochytrium* (violaxanthine) et *Scenedesmus* (lutéine).

Enfin, l'invention vise l'utilisation des microalgues déposées à la CCAP (*Culture Collection of Algae and Protozoa,* Oban, Ecosse) sous les numéros d'accession indiqués ci-après, pour enrichir des mollusques filtreurs et plus particulièrement des huîtres en acides gras polyinsaturés et/ ou en caroténoïdes selon le procédé de l'invention :
- *Schizochytrium* : CCAP n°4087/1,
- *Odontella* : CCAP n°1054/5 ;
- *Monodus sp.* : CCAP n°848/3 ;
- *Isochrysis* : CCAP n°927/16.
- *Euglena gracilis* : CCAP n°1224/49 ;
- *Cyclotella cryptica* : CCAP n°1070/7 ;
- *Nitzschia brevirostris* : CCAP n°1052/21 ;
- *Scenedesmus* : CCAP n°276/75.

Selon un exemple non limitatif de l'invention, la stabulation des huîtres se fait dans des bassins en ciment dont la dimension est d'environ 20 m x 10 m pour 1,50 m de profondeur. Les microalgues sont produites séparément en laboratoire par hétérotrophie ou mixotrophie, dans des bioréacteurs de 30 litres avec agitation puis transportés en cubiténaires de 20 litres dans lesquels les microalgues sont concentrées à raison de 60 g à 120 g de matière sèche par litre. La souche de *Schizochytrium* a été cultivée dans un fermenteur 30 L dans un milieu de culture riche [Bahnweg, G. (1979) Studies on the physiology of Thraustochytriales I. Growth requirements and nitrogen nutrition of Thraustochytrium spp., Schizochytrium sp., Japonochytrium sp., Ulkenia spp. and Labyrinthuloides spp. Veroff. Inst. Meeresforsch. Bremerh. 17: 245-268] à pH constant. Après environ 144 heures de fermentation, 80 g/l de biomasse peuvent être obtenus (poids sec) pour des cellules avec un contenu lipidique d'environ 45 %, dont le DHA représente environ 50 % parmi les triglycérides.

Les microalgues sont directement versées dans les bassins remplis d'eau de mer, en présence des mollusques, pour une phase d'affinage comprise entre 2 jours et 60 jours selon la concentration en nutriments recherchée. Les microalgues sont alors apportées à raison d'environ 10⁴ cellules/mL d'eau de mer.

L'invention a également pour objet une huître obtenue selon le procédé d'enrichissement selon l'invention, conformément à la revendication indépendante 9, dont la concentration en EPA et/ou DHA est supérieure à celle des huîtres élevées selon les méthodes traditionnelles, à savoir :
- une teneur en DHA supérieure à 100 mg/100g, de préférence supérieure à 120 mg/100g, et plus préférentiellement supérieure à 150 mg/100g en poids sec, et/ou
- une teneur en EPA supérieure à 200 mg/100g, de préférence supérieure à 220 mg/100g, plus préférentiellement supérieure à 250 mg/100g en poids sec, les teneurs étant indiqués pour 100 g de poids sec en chair crue.

Les teneurs en acides gras indiquées dans la présente demande sont déterminées, de préférence, par la méthode dite de Folch par chromatographie en phase gazeuse des esters méthyliques d'acides gras suivant les recommandations de l'AFNOR (NF EN ISO 5509 et NF EN ISO 5508).

L'invention sera mieux définie à la lumière des exemples qui suivent, donnés à titre illustratif seulement.

### Exemple 1 : Enrichissement d'une huître par stabulation en présence de la microalgue Nitzschia brevirostris

### 1. Sélection de la microalgue d'intérêt

Une culture de *Nitzschia brevirostris* a été réalisée dans un bioréacteur de 2 litres avec automate dédié et supervision par station informatique. Le système est régulé en pH via l'ajout d'une base (solution d'hydroxyde de sodium à 1N) et/ou d'acide (solution d'acide sulfurique à 1N).

La température de la culture est fixée à 23°C, et la culture est soumise à une agitation contrôlée.

Le bioréacteur est équipé d'un système d'éclairement externe entourant la cuve transparente. Ce système d'éclairement externe est composé de lampes LED réparties autour de la paroi externe de la cuve du bioréacteur. L'intensité ainsi que les cycles de lumière sont contrôlés par l'automate. La culture est alimentée avec du glucose à une concentration entre 100 mM et 150 mM. La culture est effectuée pendant au moins 7 jours, de préférence de 7 à 10 jours.

La culture est illuminée avec 30 flashs par heure, chaque flash ayant une durée de 30 secondes et une intensité de 80 µmol.m⁻².s⁻¹.

On sélectionne ainsi des microalgues d'intérêt de la souche *Nitszchia brevirostris* capables de croître en conditions de culture mixotrophe et dont la teneur en acides gras polyinsaturés, en particulier EPA et DHA, et en caroténoïdes, en particulier la fucoxanthine, est significativement plus importante qu'une souche de *Nitszchia brevirostris* non sélectionnée.

A titre d'exemple, une souche de *Nitszchia brevirostris* ainsi sélectionnée peut être la souche FCC810 déposée auprès de la CCAP *(Culture Collection of Algae and Protozoa,* Oban, Ecosse) sous le numéro d'accession CCAP 1052/21.

### 2. Enrichissement des huîtres par la microalgue Nitzschia brevirostris sélectionnée

Les huîtres de l'espèce *Crassostrea gigas* sont mises en stabulation dans un bassin d'eau de mer et la microalgue *Nitzschia brevirostris* sélectionnée au point 1 est ajoutée à hauteur de 6 g de matière sèche par litre et par 24h.

Les huîtres sont stockées dans des poches en maille plastique, à hauteur d'une trentaine d'huîtres par poche. Le volume d'eau contenu dans le bassin d'eau de mer est de 1 à 100m³. 2 à 3 poches sont disposées par m³ d'eau de mer. L'expérience est répétée 5 fois (5 lots) dans des conditions identiques.

### 3. Résultats

Les méthodes d'extraction sélective des lipides dont l'EPA et le DHA sont connues de l'homme du métier et sont, par exemple, décrites par Bligh, E.G. et Dyer, W.J. (1959) (« A rapid method of total lipid extraction and purification », Can. J. Biochem. Physiol., 37:911-917) ou par McCreary DK, Kossa WC, Ramachandran S, Kurtz RR. (1978) ("A novel and rapid method for the preparation of methyl esters for gas chromatography: application to the determination of the fatty acids of edible fats and oils", J Chromatogr Sci. 1978 Aug 10;16(8):329-31).

Les méthodes d'extraction et d'analyse des caroténoïdes sont également connues de l'homme du métier et sont, par exemple, décrites par S.W. Wright, S.W. Jeffrey, R.F.C. Mantoura, C.A. Llewellyn, T. Bjornland, D. Repeta, N. Welschmeyer : Improved HPLC method for the analysis of chlorophylls and carotenoids from marine phytoplankton. Marine ecology progress series : Vol. 77 : 183-196, 1991.

Les résultats indiqués dans le tableau ci-dessous sont donnés en mg d'acides gras par gramme (mg/g) de chair crue en poids sec de *Crassostrea gigas.*

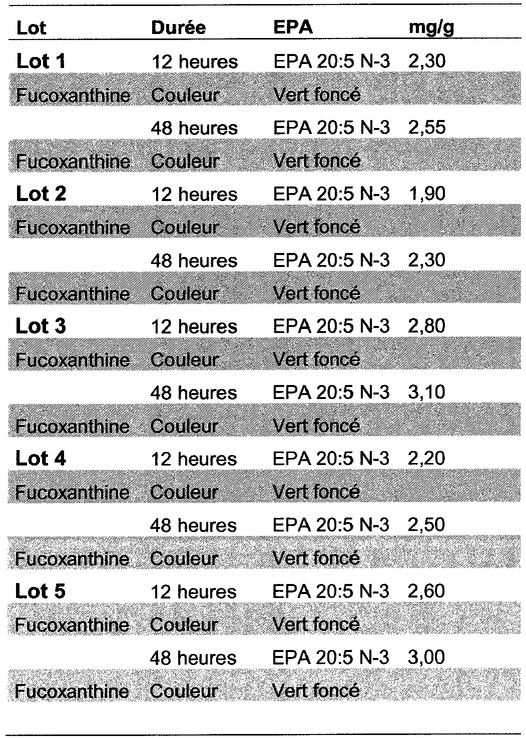

### Exemple 2 : Enrichissement d'une huître par stabulation en présence de la microalgue Schizochytrium mangrovei

### 1. Sélection de la microalgue d'intérêt

Une culture de *Schizochytrium mangrovei* a été réalisée dans un bioréacteur de 2 litres avec automate dédié et supervision par station informatique. Le système est régulé en pH via l'ajout d'une base (solution d'hydroxyde de sodium à 1N) et/ou d'acide (solution d'acide sulfurique à 1N).

La température de la culture est fixée à 23°C, et la culture est soumise à une agitation contrôlée.

Le bioréacteur est équipé d'un système d'éclairement externe entourant la cuve transparente. Le système d'éclairement externe est composé de lampes LED réparties autour de la paroi externe de la cuve du bioréacteur. L'intensité ainsi que les cycles de lumière sont contrôlés par l'automate dédié. La culture est alimentée avec du glucose à une concentration entre 30 mM et 1 M. La culture est effectuée pendant au moins 7 jours, de préférence de 7 à 10 jours.

La culture est illuminée par 30 flashs par heure, chaque flash ayant une durée de 30 secondes et une intensité de 80 µmol. m⁻².s⁻¹.

On sélectionne ainsi des microalgues d'intérêt de la souche *Schizochytrium mangrovei* capables de croître en conditions de culture mixotrophe et dont la teneur en acides gras polyinsaturés, en particulier EPA et DHA, et en caroténoïdes, en particulier l'astaxanthine, est significativement plus importante qu'une souche de *Schizochytrium mangrovei* non sélectionnée.

A titre d'exemple, une souche de *Schizochytrium mangrovei* ainsi sélectionnée peut être la souche FCC1104 déposée auprès de la CCAP (*Culture Collection of Algae and Protozoa,* Oban, Ecosse) sous le numéro d'accession CCAP 4087/1.

### 2. Enrichissement des huîtres par la microalgue Schizochytrium mangrovei sélectionnée

Les huîtres de l'espèce *Crassostrea gigas* sont mises en stabulation dans un bassin d'eau de mer et la microalgue *Schizochytrium mangrovei* sélectionnée au point 1 est ajoutée à hauteur de 6 g de matière sèche par litre et par 24h.

Les huîtres sont stockées dans des poches en maille plastique, à hauteur d'une trentaine d'huîtres par poche. Le volume d'eau contenu dans le bassin d'eau de mer est de 1 à 100m³. 2 à 3 poches sont disposées par m³ d'eau de mer. L'expérience est répétée 5 fois (5 lots) dans des conditions identiques.

### 3. Résultats

Les méthodes d'extraction sélective des lipides dont l'EPA et le DHA sont connues de l'homme du métier et sont, par exemple, décrites par Bligh, E.G. et Dyer, W.J. (1959) (« A rapid method of total lipid extraction and purification », Can. J. Biochem. Physiol., 37:911-917) ou par McCreary DK, Kossa WC, Ramachandran S, Kurtz RR. (1978) ("A novel and rapid method for the preparation of methyl esters for gas chromatography: application to the determination of the fatty acids of edible fats and oils", J Chromatogr Sci. 1978 Aug 10;16(8):329-31).

Les méthodes d'extraction et d'analyse des caroténoïdes sont également connues de l'homme du métier et sont, par exemple, décrites par S.W. Wright, S.W. Jeffrey, R.F.C. Mantoura, C.A. Llewellyn, T. Bjornland, D. Repeta, N. Welschmeyer : Improved HPLC method for the analysis of chlorophylls and carotenoids from marine phytoplankton. Marine ecology progress series : Vol. 77 : 183-196, 1991.

Les résultats indiqués dans le tableau ci-dessous sont donnés en mg d'acides gras par gramme (mg/g) de chair crue en poids sec de *Crassostrea gigas.*

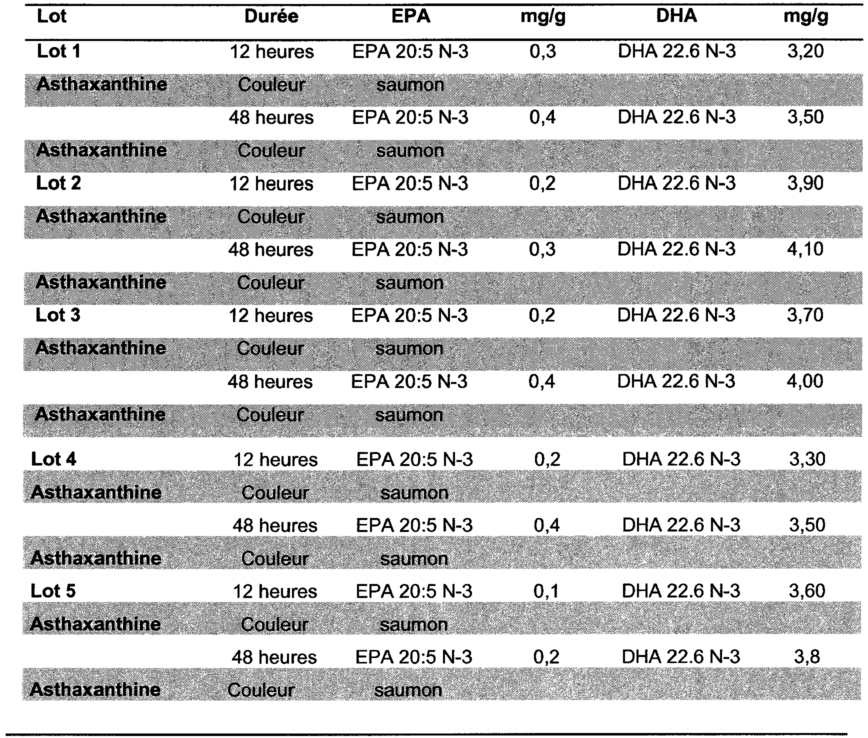

## Revendications

1. Procédé d'enrichissement d'huitres en acides gras polyinsaturés, ces acides gras polyinsaturés comprenant du DHA, de l'EPA et/ou de l'ARA, et/ou en caroténoïde(s) choisis parmi l'astaxanthine, la lutéine, la fucoxanthine, la zéaxanthine et/ou la violaxanthine, comprenant les étapes suivantes :
- la mise en stabulation des huitres dans un bassin d'eau de mer, et
- l'ajout de microalgues d'intérêt dans ledit bassin d'eau de mer, lesdites microalgues d'intérêt étant préalablement sélectionnées et cultivées de sorte à contenir au moins 2 % en poids sec d'acides gras polyinsaturés mentionnés ci-dessus et/ou au moins 1 % en poids sec d'au moins un des caroténoïdes mentionnés ci-dessus, le procédé étant réalisé dans les 1 à 10 semaines précédant la consommation desdites huitres.

2. Procédé selon la revendication 1, dans lequel lesdites microalgues d'intérêt
consistent en un mélange de différentes espèces.

3. Procédé selon l'une quelconque des revendications 1 ou 2, dans lequel les microalgues d'intérêt sont ajoutées dans le bassin d'eau de mer sous la forme d'une culture concentrée.

4. Procédé selon l'une quelconque des revendications 1 à 3, dans lequel la concentration cellulaire en microalgues dans le bassin d'eau de mer est d'au moins 10⁴ cellules par millilitre.

5. Procédé selon l'une quelconque des revendications 1 à 4, dans lequel les microalgues d'intérêt sont sélectionnées parmi les espèces des genres suivants : *Schizochytrium, Thraustochytrium, Odontella, Phaeodactylum, Nanochloris, Crypthecodinium, Monodus, Nannochloropsis, Isochrysis, Euglena, Cyclotella, Nitzschia, Aurantiochytrium, Scenedesmus* et/ou *Tetraselmis.*

6. Procédé selon la revendication 5, dans lequel les microalgues d'intérêt sont sélectionnées parmi les espèces des genres suivants : *Schizochytrium, Thraustochytrium, Odontella, Phaeodactylum, Nanochloris, Crypthecodinium, Monodus* et/ou *Nannochloropsis.*

7. Procédé d'affinage d'une huître de consommation comportant le procédé selon l'une des revendications 1 à 6, l'affinage de l'huître s'effectuant dans le bassin d'eau de mer en présence desdites microalgues d'intérêt, pendant une durée suffisante pour que ladite huître fixe les acides gras polyinsaturés et/ou le(s) caroténoïde(s) contenu(s) dans lesdites microalgues.

8. Procédé selon la revendication 7, dans lequel la durée d'affinage de l'huître est de deux jours à dix semaines.

9. Huître obtenue selon le procédé selon l'une quelconque des revendications 1 à 8, **caractérisée en ce qu'**elle a une teneur en DHA supérieure à 100 mg/100gen poids sec, et/ou une teneur en EPA supérieure à 200 mg/100gen poids sec, les teneurs étant indiquées pour 100 g de poids sec en chair crue.

## Patentansprüche

1. Verfahren zur Anreicherung von Austern mit mehrfach ungesättigten Fettsäuren, wobei diese mehrfach ungesättigten Fettsäuren DHA, EPA und/oder ARA umfassen, und/oder mit Carotinoid(en), ausgewählt aus dem Astaxanthin, dem Lutein, dem Fucoxanthin, dem Zeaxanthin und/oder dem Violaxanthin, umfassend die folgenden Schritte:
- Einstallung der Austern in ein Meerwasserbecken, und
- Hinzufügen von entsprechenden Mikroalgen in das Meerwasserbecken, wobei die entsprechenden Mikroalgen zuvor derart ausgewählt und kultiviert wurden, dass sie mindestens 2% Trockenmasse oben erwähnte mehrfach ungesättigte Fettsäuren und/oder mindestens 1% Trockenmasse mindestens eines der oben erwähnten Carotinoide enthalten,
wobei das Verfahren in den 1 bis 10 Wochen vor dem Verzehr der Austern durchgeführt wird.

2. Verfahren nach Anspruch 1, wobei die entsprechenden Mikroalgen aus einem Gemisch unterschiedlicher Spezies bestehen.

3. Verfahren nach einem der Ansprüche 1 oder 2, wobei die entsprechenden Mikroalgen in das Meerwasserbecken in Form einer konzentrierten Kultur hinzugefügt werden.

4. Verfahren nach einem der Ansprüche 1 bis 3, wobei die zelluläre Mikroalgenkonzentration in dem Meerwasserbecken mindestens 10⁴ Zellen je Milliliter beträgt.

5. Verfahren nach einem der Ansprüche 1 bis 4, wobei die entsprechenden Mikroalgen aus den Spezies der folgenden Arten ausgewählt sind: *Schizochytrium, Thraustochytrium, Odontella, Phaeodactylum, Nanochloris, Crypthecodinium, Monodus, Nannochloropsis, Isochrysis, Euglena, Cyclotella, Nitzschia, Aurantiochytrium, Scenedesmus* und/oder *Tetraselmis.*

6. Verfahren nach Anspruch 5, wobei die entsprechenden Mikroalgen aus den Spezies der folgenden Arten ausgewählt sind: *Schizochytrium, Thraustochytrium, Odontella, Phaeodactylum, Nanochloris, Crypthecodinium, Monodus* und/oder *Nannochloropsis.*

7. Verfahren zur Veredlung einer für den Verzehr vorgesehenen Auster, umfassend das Verfahren nach einem der Ansprüche 1 bis 6, wobei die Veredlung der Auster in dem Meerwasserbecken bei Anwesenheit der entsprechenden Mikroalgen während einer Dauer stattfindet, die ausreichend ist, dass die Auster die mehrfach ungesättigten Fettsäuren und/oder das/die in den Mikroalgen enthaltene(n) Carotinoid(e) bindet.

8. Verfahren nach Anspruch 7, wobei die Dauer der Veredelung der Auster zwei Tage bis zehn Wochen beträgt.

9. Auster, erhalten gemäß dem Verfahren nach einem der Ansprüche 1 bis 8, **dadurch gekennzeichnet, dass** sie einen Gehalt an DHA über 100 mg/100gen Trockenmasse und/oder einen Gehalt an EPA über 200 mg/100gen Trockenmasse hat, wobei die Gehalte für 100 g Trockenmasse rohes Fleisch angegeben werden.

## Claims

1. A method for enriching oysters in polyunsaturated fatty acids comprising DHA, EPA and/or ARA and/or carotenoid(s) chosen among astaxanthin, lutein, fucoxanthin, zeaxanthin and/or violaxanthin, comprising the following steps:
- setting the oysters in a seawater tank, and
- adding microalgae of interest to said seawater tank, said microalgae of interest being selected and cultivated beforehand so as to contain at least 2% by dry weight of polyunsaturated fatty acids mentioned above and/or at least 1% by dry weight of at least one of the carotenoids mentioned above,
the method being carried out in the 1 to 10 weeks before said molluscs are consumed.

2. The method as claimed in claims 1, wherein said microalgae of interest consist of a mixture of different species.

3. The method as claimed in any one of claims 1 or 2, wherein the microalgae of interest are added to the seawater tank in the form of a concentrated culture.

4. The method as claimed in any one of claims 1 to 3, wherein the cellular concentration of microalgae in the seawater tank is at least 10⁴ cells per milliliter.

5. The method as claimed in any one of claims 1 to 4, wherein the microalgae of interest are selected from species of the following genera: *Schizochytrium, Thraustochytrium, Odontella, Phaeodactylum, Nannochloris, Crypthecodinium, Monodus, Nannochloropsis, Isochrysis, Euglena, Cyclotella, Nitzschia, Aurantiochytrium, Scenedesmus* and/or *Tetraselmis.*

6. The method as claimed in claim 5, wherein the microalgae of interest are selected from species of the following genera: *Schizochytrium, Thraustochytrium, Odontella, Phaeodactylum, Nannochloris, Crypthecodinium, Monodus* and/or *Nannochloropsis.*

7. A method for maturing an edible oyster comprising the method as claimed in any one of claims 1 to 6, the maturing of the oyster being carried out in the seawater tank in the presence of said microalgae of interest, for a sufficient period so that said oyster fixes the polyunsaturated fatty acids and/or carotenoid(s) contained in said microalgae.

8. The method as claimed in claim 7, wherein the maturing period of the oyster is 2 days to 10 weeks.

9. The oyster obtained by the method as claimed in any one of claims 1 to 8, **characterized in that** it has a DHA content above 100 mg/100g dry weight and/or an EPA content above 200 mg/100g dry weight, the content being given for 100g dry weight of crude flesh.
